# EUROPEAN PATENT SPECIFICATION

(11) **EP 3 648 796 B1**
(45) Date of publication and mention of the grant of the patent: **05.04.2023**
(21) Application number: 17745882.5
(22) Date of filing: 07.07.2017
(51) Int. Cl.: A61K 47/10, A01N 25/00, A61K 47/36, A61K 9/10

(54) **DISPERSION FOR AN AQUEOUS SPRAY FORMULATION WITH CONTROLLED MECHANICAL DEGRADATION**
DISPERSION FÜR EINE WÄSSRIGE SPRÜHFORMULIERUNG MIT KONTROLLIERTEM MECHANISCHEM ABBAU
DISPERSION POUR UNE FORMULATION DE PULVÉRISATION AQUEUSE À DÉGRADATION MÉCANIQUE CONTRÔLÉE

(43) Date of publication of application: 13.05.2020
(73) Proprietor: GreenA B.V., 1098 XH Amsterdam (NL)
(72) Inventor: BONN, Daniel, 1098 XH Amsterdam (NL)
(74) Representative: Algemeen Octrooi- en Merkenbureau B.V.
(86) International application number: PCT/NL2017/050455
(87) International publication number: WO 2019/009697

(56) References cited:
- EP-A1- 0 128 661
- WO-A1-99/25312
- WO-A1-2016/153336
- US-A- 4 374 216

## Description

The present invention relates to a dispersion for an aqueous spray formulation with controlled mechanical degradation, to a method for controlling the mechanical degradation of a dispersion for an aqueous spray formulation, to the use of a dispersion for controlling mechanical degradation of an aqueous spray formulation, and to a method for preparing an aqueous spray formulation.

When polymers are subjected to large shear force, chain scission occurs; this phenomenon is known as mechanical degradation of the polymer; said mechanical degradation leads to a reduction of the molecular weight of the polymer, and hence to a change in the properties of the polymer.

Marherfka J., et al. Biorheology, vol. 45, 2008, 599-609, have shown that certain long-chain soluble polymers, known as drag reducing polymers, can lose their drag reducing properties due to mechanical degradation of the polymer.

From US 4,620,048 is known a mineral lubricating oil fluid containing a major amount of mineral lubricating oil and about 0.5 to 10 weight % of a soluble non-linear macromolecular polydisperse polymer as a viscosity index improver.

From EP 2 540 794 A1 is known a method comprising adding, to a solution including active polymers, an additive comprising a sacrificial polymer whose mechanical degradation is preferential with respect to the active polymers to limit the mechanical degradation of the active polymers.

From International patent application WO 2016/153336 by the present inventor is known an aqueous spray formulation comprising a polymer and a non-ionic surfactant, which provides a reduced drift and an increased deposition.

EP 0 128 661 A1 discloses a dispersion of a polysaccharide in oil that contains a dispersion promotor selected from surfactants and stabilisers. The dispersion promotors are generally selected from amphiphatic copolymers and surfactants having HLB at 20°C above 6.5 but which serve as water in oil dispersing agents during the formation of the dispersion.

WO 99/25312 A1 discloses stable fluidized polymer suspensions containing cationic polysaccharide, stabilizing agent and emollient. The preferred cationic polysaccharides are cationic guar and cationic hydroxypropyl guar, and preferred emollients are hydrocarbons, silicone oils and esters.

US 4 374 216 A discloses non-gelling water-in-oil high solids slurry dispersion of water-soluble polymer. The slurry dispersion composition contains between about 40-80 weight percent of water-soluble polymer.

Dispersions for an aqueous spray formulation comprising a polymer composition are desired, which have controlled mechanical degradation.

It is an aim of the present invention to provide a dispersion comprising a polymer composition that, upon addition to an aqueous spray formulation has controlled mechanical degradation of the formulation to which it is added.

It is an aim of the present invention to provide a method for controlling mechanical degradation of a dispersion for an aqueous spray formulation, wherein said dispersion comprises a suspension of a polymer composition.

It is an aim of the present invention to provide a use of a dispersion comprising a polymer composition that, upon addition to an aqueous spray formulation, has controlled mechanical degradation of the formulation to which it is added.

It is another aim of the present invention to provide a method for preparing an aqueous spray formulation, comprising a polymer composition, wherein said aqueous spray formulation has controlled mechanical degradation.

### Summary of the invention

The present invention relates in a first aspect to a dispersion for an aqueous spray formulation with controlled mechanical degradation, said dispersion comprising a polymer composition and at least one liquid phase, wherein said polymer composition is dispersed in said at least one liquid phase, wherein said polymer composition comprises one or more water-soluble polymers, wherein said one or more water-soluble polymers is polyethylene oxide, wherein said at least one liquid phase is an oil phase and/or an aqueous phase, and wherein said at least one liquid phase comprises at least one non-ionic surfactant with a HLB of more than 10, preferably of more than 12.

The present inventor has discovered that by preparing dispersions having controlled release into an aqueous spray formulation, mechanical degradation of the polymer can be reduced and even eliminated. By "controlled release" it is understood that the polymer composition is formulated to release the polymer gradually into the aqueous spray formulation for which it may be used.

Disclosed are dispersions comprising one or more water-soluble polymer chosen from the group consisting of: polyethylene oxide, guar gum, xanthan gum, polyacrylamide, and carboxy-methyl-cellulose.

In an embodiment said at least one non-ionic surfactant is chosen from the group consisting of surfactants having a lipophilic part and an ethylene oxide-containing hydrophilic part, surfactants having a lipophilic part and a polyethylene-containing hydrophilic part, surfactants having a lipophilic part and a polysaccharide-containing hydrophilic part, and block-copolymer surfactants having a polymer lipophilic part and a polymer hydrophilic part, and combinations thereof.

In an embodiment, said lipophilic part is a hydrocarbon part, preferably comprised of either polypropylene oxide or one or more aliphatic hydrocarbon chains, preferably alkyl groups or fatty acid residue, more preferably selected from the group consisting of fatty alcohol polyglycol ethers, preferably ethoxylates, or ethylene glycol n-alkane ethers.

In an embodiment said block copolymer surfactants are surfactants having one polymer lipophilic part and one polymer hydrophilic part, preferably comprising styrene or PPO as the polymer lipophilic part, more preferably a PEO-PPO block copolymer.

In an embodiment said polymer composition is in solid form, such as a powder or a granulate.

In an embodiment said dispersion further comprises at least one active ingredient, such as a plant protection product, a dye, or a chemical compound for coating a surface.

In an embodiment, said dispersion comprises 5-70 wt.% of said at least one active ingredient, preferably 10-60 wt.%, more preferably 15-50 wt.%, based on the total weight of the dispersion.

In an embodiment, said dispersion further comprising at least one active ingredient, comprises a polymer composition, said polymer composition comprising 10-70 wt.% of said at least one active ingredient, preferably 15-60 wt.%, more preferably 15-50 wt.%, based on the total weight of the polymer composition.

In an embodiment, said polymer composition comprises 30-100 wt.% of said one or more water-soluble polymers, preferably 40-100 wt.%, more preferably 50-100 wt.%, based on the total weight of the polymer composition.

In an embodiment said dispersion is an aqueous suspension of said polymer composition in one liquid phase, wherein said liquid phase is an aqueous phase.

In an embodiment, said aqueous suspension comprises 0.001- 10 wt.% of the one or more water-soluble polymer and 0.1-15 wt.% of said non-ionic surfactant, both based on the total weight of the suspension.

In an embodiment, said dispersion comprises an oil suspension of said polymer composition in a first liquid phase, wherein said first liquid phase is an oil phase, preferably said oil phase comprising one or more vegetable oils.

In an embodiment, said suspension comprising an oil suspension dispersed in an oil phase comprises 0.001-10 wt.% of the one or more water-soluble polymer, and said oil phase comprises 0.1-15 wt.% of said non-ionic surfactant, both based on the total weight of the suspension.

In an embodiment, said dispersion comprising an oil suspension dispersed in an oil phase further comprises a second liquid phase, said suspension of said polymer composition in a first liquid phase being further dispersed in said second liquid phase, said second liquid phase being an aqueous phase, and said dispersion being a suspoemulsion of said suspension of said polymer composition in an oil phase dispersed in an aqueous phase.

In an embodiment of the suspoemulsion, said aqueous phase comprises 0.1-15 wt.% of said at least one non-ionic surfactant, based on the total weight of the aqueous phase.

In an embodiment, said suspoemulsion comprises 10-40 wt.% of said suspension, preferably 15-35 wt.%, more preferably 20-30 wt.%, based on the total weight of the suspoemulsion.

The present invention relates in a second aspect to a method for controlling the mechanical degradation of a dispersion, said dispersion being for an aqueous spray formulation, said dispersion comprising a suspension of a polymer composition dispersed in at least one liquid phase, wherein said polymer composition comprises one or more water-soluble polymers, wherein said one or more water-soluble polymers is polyethylene oxide, wherein said at least one liquid phase is an oil phase and/or an aqueous phase, wherein said at least one liquid phase comprises at least one non-ionic surfactant with a HLB of more than 10, preferably of more than 12; and wherein said method comprises the steps of:
i) providing the polymer composition,
ii) preparing the at least one liquid phase by mixing the at least one non-ionic surfactant with said at least one liquid phase,
iii) preparing the suspension by dispersing the polymer composition of step i) in the at least one liquid phase of step ii).

In an embodiment of the second aspect, the liquid phase of step ii) is an aqueous phase. In an embodiment of the second aspect, said liquid phase of step ii) is a first liquid phase, said first liquid phase being an oil phase.

In an embodiment of the second aspect, said method further includes steps:
i) preparing a second liquid phase, said second liquid phase being an aqueous phase, by mixing at least one non-ionic surfactant with water,
ii) preparing a suspoemulsion by dispersing the suspension of step iii) in the aqueous phase of step iv).

The present invention relates in a third aspect to the use of the dispersion according to the present invention for controlling mechanical degradation of an aqueous spray formulation.

The present invention relates in another aspect to a method for preparing an aqueous spray formulation by mixing the dispersion according to the present invention and water in a weight ratio of between 1:50 to 1:1000.

### Definitions

The following definitions are used in the present application.
"polymer composition" as used in the present application means: a composition that comprises one or more water soluble polymers and optionally other materials. This composition can be used as adjuvant in the preparation of an aqueous spray formulation suitable, for example, for agricultural use, for spray painting, etc. Adjuvants are materials that are added to a spray formulation to increase the effectiveness or change the properties of the active or relevant ingredient in the spray formulation.
"mechanical degradation" as used in the present application means: change in the properties of a formulation comprising a polymer composition due to chain scission of the polymer, when this is subjected to large shear forces, e.g. spraying, shearing.
"controlled release" as used in the present application means: polymer composition that formulated to release the polymer gradually into the aqueous spray formulation.
"formulation" as used in the present application means: an aqueous spray formulation that is used for spraying, said formulation comprising water, at least one active ingredient and one or more additives.
"surfactant" as used in the present application means: a compound that, when present in water, lowers the surface tension of the water, preferably an amphiphilic compound that comprises a hydrophilic part and a lipophilic part.
"suspension" as used in the present application means: a dispersion of a solid in a liquid; wherein said solid is insoluble or has reduced solubility in the dispersing liquid.
"suspoemulsion" as used in the present application means: a dispersion of a suspension in a liquid to form an emulsion; wherein the continuous phase of the suspension (e.g. one or more oils) is immiscible with the continuous phase of the emulsion (e.g. an aqueous solution such as water).
"powder" as used in the present application means: material composed of very fine particles that are not cemented together, wherein said particles have an average particle diameter, according to ISO 9276-2 (2014), of less than 0.2 mm.
"granulate" as used in the present application means: multiparticle entity formed by making primary powder particles to adhere, in order to form larger particles. These larger particles have an average particle diameter, according to ISO 9276-2 (2014), of between 0.2 and 4.0 mm.
"treatment composition" as used in the present application means: a composition comprising an active ingredient and adjuvants. Said treatment composition being suitable in the preparation of an aqueous spray formulation suitable, for example, for agricultural use, for spray painting, for coating a surface, etc.
"active ingredient" as used in the present application means: a chemical compound that is designed to achieve a specific effect; for example, a dye for spray painting a surface; a chemical compound for coating a surface; a chemical compound (e.g. plant protection product) for control or elimination of a particular pest in the agricultural industry and/or for helping or stimulating growth of a plant, such as a pesticide (e.g. a herbicide, an insecticide, a fungicide, a nematicide, a molluscicide, a miticide), a plant growth regulator, a defoliant, a growth stimulator, or a nutrient.
"plant protection product" as used in the present application means: a product for agricultural use, said product being used for stimulating or promoting plant growth and/or controlling or eliminating a specific pest. Examples of pant protections products are: a plant growth regulator, a defoliant, a growth stimulator, a nutrient, a pesticide, or other product designed to control or eliminate a particular pest and/or help stimulate the growth of a plant comprising one or more active ingredients.
"effective amount" as used in the present application means: the relative amount of the active ingredient that is effective to, for example, achieve complete coverage of a surface, achieve a desired degree of coating of a surface, or control a pest or to promote plant growth when the formulation is spray applied to the surface or plant and/or to the environment of the plant at a given application rate.
"droplet" as used in the present application means: a droplet of an aqueous spray formulation in the spray.
"volume % of droplets < 100 micrometer" as used in the present description is a measure of the fraction of fine droplets, viz. droplets having a diameter of < 100 micrometer in the total spray. It is expressed as a percentage of the total volume of all droplets in a spray.

In the present application, when making reference to a range in the form "between a-b" or just "a-b", it means that said range includes both values a and b and all values in between these two values.

### Detailed description of the invention

The present invention is discussed in more detail below.

The present invention relates to a dispersion comprising a polymer composition dispersed in at least one liquid phase; wherein said polymer composition comprises one or more water-soluble polymers being polyethylene oxide, which are insoluble or have reduced solubility in the at least one liquid phase. This dispersion has a controlled mechanical degradation when the dispersion is added to an aqueous spray formulation and this formulation is sprayed.

The present invention relates in a first aspect to a dispersion for an aqueous spray formulation with controlled mechanical degradation, said dispersion comprising a polymer composition and at least one liquid phase, wherein said polymer composition is dispersed in said at least one liquid phase, wherein said polymer composition comprises one or more water-soluble polymers, wherein said one or more water-soluble polymers is polyethylene oxide, wherein said at least one liquid phase is an oil phase and/or an aqueous phase, and wherein said at least one liquid phase comprises at least one non-ionic surfactant with a HLB of more than 10, preferably of more than 12.

This dispersion may also be called a tank-mix composition since it may be added to a spray tank filled with water in order to obtain the aqueous spray formulation comprising at least one active ingredient and one or more additives. The active ingredient can be added directly to the water and/or can be added within the dispersion prior or during spraying of the aqueous spray formulation. This active ingredient can be, for example, a dye, a chemical compound for coating a surface, a plant protection product, etc.

The dispersion according to the invention can be used as an adjuvant or carrier of an active ingredient. The dispersion according to the invention provides for a dispersion that has controlled mechanical degradation, which, for example, can be particularly useful in the agricultural industry. In this case, the dispersion according to the invention can be used as adjuvant that improves the spraying of substances for plant protection or nutrition products. Such products can be used for stimulating or promoting plant growth and/or controlling or eliminating a specific pest. The product needs to be applied to the (plants on the) area, for example by spraying. A spraying operation is generally conducted either from the air - aerial application using e.g. spray planes - or from the ground - ground application using e.g. crop spraying machines - depending on the situation. Ground spraying also includes hand-held refillable pressurized tank sprayers. Conventional equipment is available for either type of application.

In a first embodiment the dispersion comprises an aqueous suspension of the polymer composition in an aqueous phase, said polymer composition comprising one or more water-soluble polymers and said aqueous phase comprising at least one non-ionic surfactant with a HLB of more than 10, preferably or more than 12. Preferably said non-ionic surfactant has a HLB of at most 25. Preferably, the aqueous suspension comprises 0.001- 10 wt.% of the one or more water-soluble polymer and 0.1-15 wt.% of said non-ionic surfactant, both based on the total weight of the suspension.

The one or more non-ionic surfactants present in the aqueous phase of the suspension significantly reduce the solubility of the one or more water-soluble polymers in the aqueous phase; therefore, an aqueous suspension of the polymer composition comprising said one or more water-soluble polymers is obtained.

In a second embodiment the dispersion comprises an oil suspension of the polymer composition comprising one or more water-soluble polymers in an oil phase, preferably said oil phase comprising an oil chosen from the group consisting of vegetable oils and mineral oils; wherein said oil phase comprises at least one non-ionic surfactant with a HLB of more than 10, preferably of more than 12. Preferably said non-ionic surfactant has a HLB of at most 25. Preferably, the oil suspension comprises 0.001- 10 wt.% of the one or more water-soluble polymer, and the oil phase comprises 0.1-15 wt.% of said non-ionic surfactant, both based on the total weight of the suspension.

The one or more non-ionic surfactants present in the oil phase of the suspension help stabilizing the oil suspension of the polymer composition comprising said one or more water-soluble polymers.

In both the first (aqueous phase) embodiment and the second (oil phase) embodiment, a dispersion is obtained (respectively an aqueous suspension and an oil suspension), which upon addition thereof to an aqueous spray formulation has a controlled mechanical degradation when the aqueous formulation is sprayed. This effect is obtained, because in both cases a dispersion is obtained, which has a controlled release of the polymer composition upon addition to an aqueous spray formulation.

In a third embodiment the suspension of a polymer composition dispersed in a first liquid phase, said first liquid phase being an oil phase, viz. the oil suspension, is further dispersed in a second liquid phase; by doing this a suspoemulsion is formed. In this embodiment, the second liquid phase of said dispersion is an aqueous phase, and the dispersion is then a suspoemulsion of the suspension of said polymer composition in an oil phase dispersed in an aqueous phase. Preferably the second liquid phase, i.e. the aqueous phase of the suspoemulsion comprises 0.1-15 wt.% of said at least one non-ionic surfactant, based on the total weight of the aqueous phase.

By further dispersing the oil suspension in a liquid phase a suspoemulsion is obtained, which has a delayed release of the polymer composition upon addition to an aqueous formulation, by a slow incorporation of the polymer into the aqueous phase.

The present invention relates to a dispersion comprising a polymer composition suspended in at least one liquid phase. In an embodiment, the dispersion comprises 0.01-15 wt.% of said polymer composition and 85-99.9 wt.% of said liquid phase, both based on the combined weight of the polymer composition and the liquid phase. The present invention relates to a dispersion comprising a polymer composition suspended in at least one liquid phase. In an embodiment, the dispersion comprises 0.01-15 wt.% of said polymer composition and 85-99.9 wt.% of said liquid phase, both based on the total weight of the dispersion. In an embodiment, the combined amount of polymer composition and liquid phase amounts to 100 wt.%; in other words, in this embodiment, the invention relates to a dispersion consisting of a polymer composition suspended in a liquid phase.

The suspoemulsion can comprise 10-40 wt. % of the suspension, preferably 15-35 wt.%, more preferably 20-30 wt.%, based on the total weight of the suspoemulsion. In this embodiment of the dispersion according to the present invention, the liquid phase consists of an oil phase and of an aqueous phase. In this embodiment, the liquid phase may comprise between 10-80 wt.% of said oil phase and between 20-90 wt.% of said aqueous phase, based on the weight of said liquid phase. In this embodiment, the combined amounts of oil phase and aqueous phase may amount to 100 wt.% of said liquid phase of the dispersion.

In an embodiment the polymer composition is in solid form, such as a powder or a granulate.

In an embodiment, the dispersion according to the invention comprises at least one active ingredient such as a plant protection product, a dye, or a chemical compound for coating a surface. This active ingredient can be present in the at least one liquid phase and/or the polymer composition. An active ingredient may be present in the polymer composition and the weight thereof then falls under the weight of the polymer composition; this is a preferred embodiment. In another embodiment, the active ingredient may be present in the dispersion but not as part of the polymer composition but as part of the liquid phase and the weight thereof then falls under the weight of the liquid phase.

In an embodiment, the dispersion according to the invention comprises 5-70 wt.% of said at least one active ingredient, preferably between 10-60 wt.%, more preferably 15-50 wt.%, based on the total weight of the dispersion.

In an embodiment, the dispersion according to the invention comprises a polymer composition, in which said polymer composition - in addition to said water-soluble polymer - further comprises at least one active ingredient, such as a plant protection product, a dye, or a chemical compound for coating a surface. Preferably said polymer composition comprises 10-70% wt.% of said at least one active ingredient, preferably 15-60 wt.%, more preferably 15-50 wt.%, based on the total weight of the polymer composition. In this embodiment, the combined amount of said water soluble polymer and said one or more active ingredients form 100 wt.% based on the weight of said polymer composition.

In an embodiment, the dispersion according to the invention comprises a polymer composition, in which said polymer composition comprises 30-100 wt.% of said one or more water-soluble polymers, preferably 40-100 wt.%, more preferably 50-100 wt.%, based on the total weight of the polymer composition. In an embodiment, the amount of water-soluble polymer is at most 90 wt.%, such as at most 85 wt.% based on the total weight of the polymer composition.

In another aspect, the present invention relates to a method for controlling the mechanical degradation of a dispersion, said dispersion being for an aqueous spray formulation, said dispersion comprising a suspension of a polymer composition dispersed in at least one liquid phase, wherein said polymer composition comprises one or more water-soluble polymers, wherein said one or more water-soluble polymers is polyethylene oxide, wherein said at least one liquid phase is an oil phase and/or an aqueous phase, wherein said at least one liquid phase comprises at least one non-ionic surfactant with a HLB of more than 10, preferably of more than 12; and wherein said method comprises the steps of:
i) providing the polymer composition,
ii) preparing the at least one liquid phase by mixing the at least one non-ionic surfactant with said at least one liquid phase,
iii) preparing the suspension by dispersing the polymer composition of step i) in the at least one liquid phase of step ii).

In an embodiment the at least one non-ionic surfactant has a HLB of at most 25. In an embodiment, the liquid phase of step ii) of the method is an aqueous phase. In another embodiment, the liquid phase of step ii) is a first liquid phase, said first liquid phase being an oil phase.

The method for controlling the mechanical degradation of a dispersion according to the invention produces a polymer composition that is dispersed in liquid phase, in which said polymer composition is not soluble or has a reduced solubility. This implies that when the dispersion is added to an aqueous spray formulation, the polymer composition is not immediately dissolved in the aqueous formulation, but instead, its availability is controlled, i.e. it has controlled release. Thanks to this controlled release of the polymer composition, mechanical degradation of the polymer composition is reduced or even eliminated.

In an embodiment, the previous method for controlling the mechanical degradation of a dispersion includes further steps:
iv) preparing a second liquid phase, said second liquid phase being an aqueous phase, by mixing at least one non-ionic surfactant with water,
v) preparing a suspoemulsion by dispersing the suspension of step iii) in the aqueous phase of step iv).

In an embodiment the second liquid phase, being the aqueous phase, comprises between 0.1-15 wt.% of the at least one surfactant, based on the total weight of the continuous aqueous phase. In this embodiment, the aqueous phase comprises between 85 and 99.9 wt.% of an aqueous solution, preferably water.

In an aspect, the present invention relates to the use of the dispersion according to the invention for controlling mechanical degradation of an aqueous spray formulation.

In another aspect, the present invention relates to a method for preparing an aqueous spray formulation by mixing the dispersion according to the invention and water in a weight ratio of between 1:50 to 1:1000.

The dispersion according to the present invention may be added to the final formulation in the field prior to spraying or it may be added with the active ingredient to prepare a steady premix (treatment composition).

The dispersion according to the present invention may be a concentrate consisting of a polymer composition - consisting of one or more water soluble polymers and optionally additives- suspended in a liquid phase.

The dispersion according to the present invention may also be a treatment composition consisting of a polymer composition - consisting of one or more water soluble polymers, one or more active ingredients and optionally additives- suspended in a liquid phase.

The dispersion according to the present invention may also be a spray formulation comprising of an oil suspension of polymer composition - consisting of one or more water soluble polymers, one or more active ingredients and optionally additives that is diluted with water in a ratio of at least 1:50.

The dispersion according to the present invention may also be a spray formulation comprising of an aqueous suspension of polymer composition - consisting of one or more water soluble polymers, one or more active ingredients and optionally additives that is diluted with water in a ratio of at least 1:50

The dispersion according to the present invention may also be a spray formulation comprising of an oil-in-water suspoemulsion of polymer composition - consisting of one or more water soluble polymers, one or more active ingredients and optionally additives that is diluted with water in a ratio of at least 1:50.

In an embodiment the present invention relates to a treatment composition for a spray formulation with controlled mechanical degradation, said treatment composition comprising a suspension of a polymer composition dispersed in a liquid phase and at least one plant protection product, or dye, or a chemical compound for surface coating; wherein said polymer composition comprises one or more water-soluble polymers, and wherein said liquid phase comprises at least one non-ionic surfactant, said at least one non-ionic surfactant having a hydrophilic-lipophilic balance (HLB) of more than 10, preferably of more than 12.

In another aspect, the present invention relates to a polymer composition comprising one or more water-soluble polymer and at least one non-ionic surfactant. Preferably said polymer composition is in solid form, preferably in the form of a granulate.

This granulate can be directly added to the aqueous spray formulation and the combination of the one or more water-soluble polymers and the at least one non-ionic surfactant will care for a slow transfer of the one or more water-soluble polymer into the aqueous spray formulation; which will lead to a controlled release of the one or more water-soluble polymers; therefore, an aqueous spray formulation with controlled mechanical degradation is also obtained.

In another embodiment, said granulate can be further dispersed in a liquid phase, said liquid phase being an aqueous phase or an oil phase.

In a further aspect, the present invention relates to a method for preparing an aqueous spray formulation by mixing the treatment composition according to the invention and water in a weight ratio of between 1:50 to 1:1000. The present invention thus also relates to a dispersion in the form of a spray formulation.

In another aspect, the present invention is also related to a method for making an aqueous ready to use plant protection formulation, comprising combining the dispersion according to the invention with water and with one or more plant protection product. This method is usually carried out at a temperature between 5 and 30 °C.

In a further aspect, the present invention relates to an aqueous spray formulation with controlled mechanical degradation, comprising the treatment composition according to the invention and water.

The following embodiments are applicable to all aspects of the present invention unless it is specifically stated that the embodiments relate to a specific aspect of the invention.

Preferably, said polyethylene oxide has a molecular weight between 5 × 10⁵ (500,000) and 10⁸ (100,000,000) g/mole; preferably, at least 1 × 10⁶ (one million), more preferably at least 2×10⁶ (two million), even more preferably, at least 3×10⁶ (three million), such as at least 3.5×10⁶ (three and a half million); preferably, at most 1×10⁷ (ten million), more preferably at most 5×10⁶ (five million), such as most 4.5×10⁶ (four and a half million).
Disclosed are aqueous spray formulation comprising one or more water-soluble polymer chosen from the group consisting of: polyethylene oxide, guar gum, xanthan gum, polyacrylamide, and carboxy-methyl-cellulose.

In these formulations, said guar gum may have a molecular weight between 1.5×10⁴ (15,000) and 8×10⁶ (eight million).

In these formulations, said xanthan gum may have a molecular weight between 1x10⁵ (100,000) and 6×10⁶ (six million).

In these formulations, said polyacrylamide may have a molecular weight between 5×10⁵ (500,000) and 2×10⁷ (twenty million).

In these formulations said carboxy-methyl cellulose may have a molecular weight of between 1x10⁵ (100,000) and 2×10⁷ (twenty million).

Preferably, the one or more water-soluble polymer is biodegradable and has good anti drift properties.

The polymer composition can be in solid form, such as a powder or a granulate. The polymer composition can comprise 30-100 wt.% of said one or more water-soluble polymers, preferably 40-100 wt.%, more preferably 50-100 wt.%, based on the total weight of the polymer composition. Moreover, the polymer composition may comprise one or more active ingredients and/or additives.

The suspension of a polymer composition comprising one or more water-soluble polymers dispersed in a liquid phase can comprise 0.001-10 wt.% of the one or more water-soluble polymers, preferably 0.01-10 wt.%, more preferably 0.1-7% wt.%, based on the total weight of the suspension. Preferably, the suspension of a polymer composition dispersed in a liquid phase comprises 0.1-15 wt.% of the non-ionic surfactant, preferably 0.5 -15 wt.%, more preferably 1-12wt.%, based on the total weight of the suspension.

In an embodiment, the liquid phase comprises between 0.1-15 wt.%, preferably 0.5-12 wt.%, more preferably 1-10 wt.% of the non-ionic surfactant; and between 85-99.9 wt.%, preferably 87-99.5 wt.%, more preferably 90-99 wt.% of liquid; both based on the total weight of said liquid phase. In this embodiment, the combined amount of said non-ionic surfactant and said liquid amounts to 100 wt.% based on the total weight of said liquid phase.

In an embodiment the at least one liquid phase is an oil phase, comprising the non-ionic surfactant and one or more oils; said one or more oils are chosen from the group consisting of vegetable oils and mineral oils, preferably vegetable oils, such as sesame oil, canola oil, sunflower oil, soybean oil, peanut oil, olive oil, corn oil, grapeseed oil, jojoba oil, cotton seed oil, almond oil, safflower oil, walnut oil, avocado oil, rice bran oil, flaxseed oil, almond oil, avocado oil, canola oil, corn oil, cottonseed oil, flax seed oil, hazelnut oil, hemp seed oil, grapeseed oil, jojoba oil, macadamia nut oil, olive oil, peanut oil, rapeseed oil, rice bran oil, safflower oil, sesame oil, soybean oil, sunflower oil, walnut oil, and combinations thereof.

In an embodiment the at least one liquid phase is an aqueous phase, comprising the non-ionic surfactant and an aqueous solvent, preferably water.

The present inventors have observed that by dispersing a polymer composition comprising one or more water-soluble polymers in at least one liquid phase; wherein said one or more water-soluble polymers are insoluble or have reduced solubility in the at least one liquid phase, the release of the polymer composition into the aqueous spray formulation is controlled. The controlled release of the polymer composition is which leads to a control of the mechanical degradation of said one or more water-soluble polymers, and hence of the aqueous spray formulation, when this is sprayed.

Without wishing to be bound by any theory, the present inventors believe that the water-soluble polymer, when added to an aqueous spray formulation, contributes a "visco-elastic" effect resulting in a shift of the droplet spectrum, so that there are fewer fine droplets (i.e., less than 100 micrometers) of the final spray product and a more narrow range of droplet sizes. Therefore, when mechanical degradation of the water-soluble polymer occurs, the amount of fewer fine droplets increases.

The volume % of droplets < 100 micrometer is an important parameter since an increase of this value would indicate mechanical degradation of the polymer composition, when added to the aqueous spray formulation. The volume percentage of fine droplets is measured using the same method as VMD50. The result of the VMD50 measurement is a curve volume % in the spray is plotted against difference diameter ranges, such as 0-20 micrometer, 20-40 micrometer, 40-60 micrometers, 60-80 micrometers, 80-100 micrometers etc. Then the volume percentages of all fractions below 100 micrometers are added to arrive at the total volume percentage of droplets below 100 micrometer. More information about the calculation thereof can be found in the handbook "Principles of Colloid and Surface Chemistry, Third Edition, Revised and Expanded" edited by Hiemenz & Rajagopalan, chapter 1, paragraph 5 "some physical characteristics of colloids", page 35, Table 1.6.

When the dispersion according to the invention is added to an aqueous formulation, the viscosity of the aqueous formulation is increased and/or, when this aqueous formulation is sprayed, the volume % of droplets < 100 is reduced as compared with the aqueous formulation without added dispersion. The present inventor has observed that when mechanical degradation of the dispersion occurs, the viscosity of the aqueous formulation comprising the dispersion according to the invention decreases and/or the volume % of droplets < 100 increases. Therefore, viscosity and volume % of droplets < 100 are important parameters when determining mechanical degradation of an aqueous formulation comprising the dispersion according to the invention.

In an embodiment the at least one non-ionic surfactant is selected from the group consisting of surfactants having a lipophilic part and an ethylene oxide-containing hydrophilic part, surfactants having a lipophilic part and a polyethylene-containing hydrophilic part, surfactants having a lipophilic part and a polysaccharide-containing hydrophilic part, and block-copolymer surfactants having a polymer lipophilic part and a polymer hydrophilic part, and combinations thereof. Preferably, the lipophilic part is a hydrocarbon part, preferably comprised of one or more aliphatic hydrocarbon chains, preferably alkyl groups or fatty acid residue, more preferably selected from the group consisting of fatty alcohol polyglycol ethers, preferably ethoxylates, or ethylene glycol n-alkane ethers.

In an embodiment, surfactants having a lipophilic part and a polysaccharide-containing hydrophilic part are selected from the group, consisting of cocamide monoethanolamines, sorbitan monostearates, and alkyl polyglucosides.

Alternatively, when the chosen at least one non-ionic surfactant is a block copolymer, said block copolymer is preferably a surfactant having one polymer lipophilic part and one polymer hydrophilic part, preferably comprising styrene or PPO as the lipophilic polymer part, more preferably a PEO-PPO block copolymer.

Examples of the block copolymer surfactants are surfactants having one lipophilic polymer part and one hydrophilic polymer part, preferably comprising styrene or PPO as the polymer lipophilic part. However, other polymer lipophilic parts may be used. Preferably, said surfactants comprise PEO or a block copolymer of PEO and PPO as the hydrophilic polymer part.

It should be noted that the so-called HLB value or Hydrophic-Lipophilic Balance is an important parameter for selecting a suitable surfactant. Preferably, the non-ionic surfactant according to the present invention has a HLB value of at least 10, preferably at least 12. In an embodiment, the HLB value of the non-ionic surfactant is at most 25, preferably at most 20, preferably at most 18.

A non-limiting example of a suitable PEO-PPO block copolymer is a commercially available surfactant from the class Synperonic^{®}, which is a polyalkylene oxide block copolymer. It is a water soluble surfactant having a high HLB value.

Another suitable example of a block copolymer surfactant is an alkoxylate such as, for example, an alcohol initiated ethylene oxide/propylene oxide block copolymer like a 4-butoxy-I -butanol initiated ethylene oxide/propylene oxide block copolymer (CAS number 99821-01-9) of which Atlas^{™} G5000 and Atlas^{™} G5002L are examples, and which are available from Croda (Edison, NJ). Atlas ^{™} G5000 and Atlas ^{™} G5002L are butyl block copolymers having a HLB of 17.

Suitable examples of surfactants having an ethylene-oxide containing hydrophilic part and a fatty acid containing lipophilic part are fatty alcohol polyglycol ethers, such as narrow range ethoxylates (NRE), or ethylene glycol n-alkane ethers, or polysorbates. As an ethylene-oxide containing surfactant, Triton^{®} may be mentioned which is commercially available, such as Triton X-100 (C₁₄H₂₂O(C₂H₄O)*ₙ*) having a hydrophilic polyethylene oxide chain (on average it has 9.5 ethylene oxide units) and an aromatic hydrocarbon lipophilic or hydrophobic group. The hydrocarbon group is a 4-(1,1,3,3-tetramethylbutyl)-phenyl group. The HLB value of Triton X-100 is 13.4.

An example of a non-ionic surfactant which is commercially available is Tween 20. This is a polysorbate-type non-ionic surfactant formed by the ethoxylation of sorbitan before the addition of lauric acid. Tween 20 has an HLB of 16.7. Tween 20 is also known as polyoxyethylene (20) sorbitan monolaurate, polysorbate 20, PEG(20)sorbitan monolaurate and Alksest TW 20.

Suitable examples of polymers comprising an aliphatic hydrocarbon lipophilic group and a polysaccharide hydrophilic group are the following: cocamide monoethanolamines, sorbitan monostearates, and alkyl polyglucosides. These have HLB values of between about 13 and 14.

Other examples of suitable non-ionic surfactants are alkoxylated alkylmethylesters, and alkoxylated alkylamines, which have HLB values of between 9 and 15.

Examples of surfactants that are often used as emulsifiers in commercially available pesticide formulations are the non-ionic Zipper^{®} and Silwet^{®} (organic silicone or trisiloxanes), and the anionic AOT (dioctyl sodium sulfosuccinate or docusate sodium). These surfactants are not suitable for use in the present invention. Trisiloxanes have HLB values about 7, the HLB value of AOT is 32.

In an embodiment of all aspects of the present invention a combination of two or more non-ionic surfactants is used wherein each of the surfactants may be independently selected from the non-ionic surfactants above. In a non-limiting example both an ethylene-oxide containing surfactant and a PEO-PPO block copolymer are present in either the dispersion for an aqueous spray formulation, treatment composition or spray formulation according to the present invention. Other combinations of surfactants may also be used.

Without wishing to be bound by any theory, the present inventors believe that the surfactants as in the present invention have a double function, firstly they facilitate the dispersion of the polymer composition in the oil phase in the suspension, or the dispersion of the suspension in the aqueous phase. Secondly, non-ionic surfactants provide a stabilizing and deposition increasing effect.

In an embodiment the present dispersion is present in the final spray formulation in an amount of between 1 to 50 and 1 to 1000 based on the total weight of the final spray formulation. In other words, 1 part of the present dispersion in 50 to 1000 parts of the final spray formulation including the dispersion. Preferably, present dispersion is present in the final spray formulation in an amount of between 1 to 100 and 1 to 750, more preferably between 1 to 150 and 1 to 500, for example, between 1 to 175 and 1 to 300, such as about 1 to 200. As a non-limiting example thereof 1 liter of the present dispersion is added to a tank together with a plant protection product and then water is added to provide a final amount of 200 liters of the aqueous spray formulation. The above ranges of the presence of the dispersion in the aqueous spray formulation may also be applied to the presence of the treatment composition in the aqueous spray formulation.

Said plant protection product may be a conventional plant protection product that is commercially available. When a certain amount of active ingredient is mentioned, this can be calculated to a certain amount of a plant protection product.

Examples of plant protection products are: a plant growth regulator, a defoliant, a growth stimulator, a nutrient, a pesticide, or other product designed to control or eliminate a particular pest or help stimulate the growth of a plant. Preferably, the pesticide is selected from the group consisting of an herbicide, an insecticide, a fungicide, a nematicide, a molluscicide, and a miticide. The amount of plant protection product will depend upon the objectives of the spraying operation and can be determined by a person skilled in the art.

In an embodiment the at least one protection product is comprised in the polymer composition. In another embodiment the plant protection product is present in an effective amount.

In an embodiment of the treatment composition, the weight ratio between one or more water-soluble polymer and the plant protection product is between 1:50 and 1:4000, more preferably between 1:50 and 1:2000, more preferably between 1:100 and 1:1000, for example between 1:200 and 1:900.

In the dispersion and/or treatment composition and/or formulation, one or more solvents may be present in addition to water, preferably selected from the group consisting of aliphatic solvents, more preferably aliphatic alcohols, such as methanol, ethanol, propanol, isopropanol, and butanol, most preferably isopropanol.

Examples of plant protection products that can be used according to the present invention are: Basta^{®}, Roundup^{®}, Spotlight^{®}, Teppeki^{®}, UNIX^{®}, Thiovit^{®}, Fungistop^{®}, Regione^{®}, Supreme^{®}, Hussar^{®}, Calliste^{®}, Citadelle^{®}, Mikado^{®}, Milagro^{®}, Parlay^{®}, Scala^{®}, Equip^{®}, Karate Zeon^{®}, Evidan^{®}, Mancozeb^{®}, and Shirlan^{®}.

The active ingredient present in the spray formulation according to the present may be present in the amount as indicated by the manufacturer of said active ingredient or may be present in the amount determined by the person skilled in the art based on the requirements. However, using the dispersion according to the present invention, the amount may be reduced by at least 5 %, more preferably at least 10 %, even more preferably at least 15%, such as at least 20% while achieving the same effect because the dispersion according to the present invention increases the efficiency of the active ingredient.

Plant protection products, which according to the present inventions are active ingredients, may be solid products, such as, wettable powders, dispersible granules, as well as liquid products such as emulsifiable concentrates, suspensions, suspo-emulsions, concentrated emulsions.

Examples of suitable additives for all aspects of the present invention are a (an)ionic surfactant, a viscosity modifier, a stabilizer, a low odor paraffin solvent, an antifoam agent, a dispersant, a disintegrant, a binder, a filler, an anti-caking agent a preservative and one or more combinations thereof. All these additives are known to a person skilled in the art and may be selected based on the conditions and desired results.

Non-limiting examples of suitable anionic surfactants are alkyl sulfates or alkyl phosphates, such as lauryl sulfates, sodium alkyl sulfosuccinates, sodium alkyl sulfates, sodium lauroyl sarcosinates, sodium myreth sulfates, sodium pareth sulfate, and sodium stearates.

In an embodiment of the aqueous spray formulation, the aqueous spray formulation comprises the dispersion according to the invention and water, wherein the weight ratio of the dispersion according to the invention and water is 1:5 to 1:500.

In an embodiment of the aqueous spray formulation, the aqueous spray formulation comprises 0.01-2 wt.% of the one or more water-soluble polymers, based on the total weight of the spray formulation.

In an embodiment of said aqueous spray formulation, the amount of water is between 70 wt.% and 99 wt.% based on the total weight of the formulation.

In an embodiment of the aqueous spray formulation, the aqueous spray formulation comprises 0.1-20 wt.% of the at least one non-ionic surfactant, based on the total weight of the spray formulation.

The dispersion according to the invention, when added to an aqueous spray formulation, leads to an aqueous spray formulation that has controlled mechanical degradation. This means that when said aqueous spray formulation is sprayed, the viscosity of the formulation does not decrease and/or the number of droplets with a size smaller than 100 µm is reduced, and this reduction remains stable in time.

The reduction in spray drift is achieved simultaneously with the same dispersion for aqueous spray formulation according to the invention that reduces significantly the amount of droplets of small size (also called fine size), which are mainly responsible for the drift.

The type of nozzle is not limited in the present invention. Examples of nozzles that are suitable for use with the aqueous spray formulation according to the present invention when used in broadcast spraying from either air or ground are flat spray nozzles providing a triangular spray. In other words, nozzles that provide a tapered edge flat spray pattern. These types of nozzles provide for uniform coverage in broadcast spraying of plants. The spray angle may vary according to the desired use and may be determined by a person skilled in the art. Examples are spray angles of 80 °, 90 °, 100 ° and 110°, such as the XR Teejet 110^{®} or nozzles from the Teejet ^{®} VisiFlow ^{®} range. They may be operated using a wide range of pressure, for example at a pressure between 2 and 5 bar.

In order to facilitate a further understanding of the invention, the following example is presented primarily for the purpose of illustrating more specific details thereof. The invention should not be deemed limited thereby.

### Materials and methods

The viscosity as specified in the present application has the unit of mPa.s and is measured at a temperature of 20 °C using an Anton Paar MCR302 rotational rheometer using a 50mm plate-plate geometry. The rotational speed is kept constant 1000 s⁻¹, and the resulting shear stress is measurement as a function of time; the viscosity is then calculated as the ratio between the shear stress and shear rate. The duration of the measurement is 17.09 seconds for each point and the shear stress is measured during a total time period of between 150 and 800 s at which time the viscosity value is determined.

The VMD50 as specified in the present application has the unit of micrometer and is measured using a laser diffraction technique using a Malvern SprayTech^{®} instrument with the spray nozzle 30 cm above the top of the laser beam. The Malvern Spraytec uses the technique of laser diffraction for measurement of the size of spray droplets and spray particles. The laser beam passes through spray and is diffracted; the diffracted beam is detected by a CCD detector that is directly opposite the laser. The diffraction pattern is then analyzed to calculate the size of the droplets and the volume of droplets and the number of droplets for each volume/size. From this, the VMD is calculated using known mathematical techniques.

The present invention is elucidated by the following examples, which are in no way limiting to the scope of the invention as defined by the appended claims.

### Examples

As specified above, the present invention is related to the control of mechanical degradation of dispersions for an aqueous spray formulation, said dispersion comprising a suspension of a polymer composition dispersed in a liquid phase. The liquid phase can be an aqueous phase or an oil phase. These dispersion can in addition be formulated as a suspoemulsion; i.e. a dispersion of a suspension in an aqueous phase. In order to show the control of mechanical degradation of said dispersion the volume % of droplets < 100 micrometer and/or the viscosity is measured as a function of time; this is done in Example 1 for aqueous suspensions, in Example 2 for an oil suspension, in Example 3 for a suspoemulsion and in Example 4 for granulates.
In all Examples shown below, the following nomenclature is used:
t_{f} = time at the end of the measurement, i.e. duration of the experiment.
η at t₀ = viscosity at the beginning of the experiment.
η at t_{f}= viscosity at the end of the experiment, i.e. at t_{f}.
V% < 100µm at t₀= V% < 100µm at the beginning of the experiment.
V% < 100µm at t_{f}= V% < 100µm at the end of the experiment, i.e. at t_{f}.

### Example 1

The composition of different aqueous suspensions according to the invention is shown in Table 1. Aqueous suspensions comprising one water-soluble polymer were prepared. The different water-soluble polymers that were used for preparing the samples are: guar gum with an average molecular weight of 2×10⁶ (two million), polyethylene oxide with an average molecular weight of 4×10⁶ (four million - PEO) and carboxy-methyl-cellulose (CMC) with an average molecular weight of 1x10⁶ (one million). The aqueous suspensions were prepared by mixing the surfactants with water to obtain an aqueous phase. Subsequently, the polymer composition was dispersed in the aqueous phase.

**Table 1**

| | A | B | C |
|---|---|---|---|
| Polymer type | Guar | PEO | CMC |
| wt. % polymer in polymer composition | 100 | 100 | 100 |
| wt. % polymer in suspension | 1.00 | 0.45 | 2 |
| wt. % synperonic ^{®} PE/F127 in suspension | 6.00 | 4.50 | 4 |
| wt. % Atlas^{™} G5002 L in suspension | 0 | 2.00 | 0 |
| wt. % Polysorbate 20 in suspension | 0 | 4.50 | 0 |
| wt. % water in suspension | 93 | 89 | 94 |
| Aqueous spray formulation g polymer composition / I water | 0.14 | 0.02 | 0.28 |

For the samples in table 1 the volume % of droplets < 100 micrometer was determined as a function of time. These results are shown in Table 2. The results were compared with solutions of the polymers in water, without any surfactant; these are the comparative examples which are marked with an "*". Examples A and C are not according to the present invention, since they do not comprise polyethylene oxide as water-soluble polymer.

**Table 2**

| Sample | t_{f} (s) | V% < 100µm at t₀ (%) | V% < 100µm at t_{f} (%) |
|---|---|---|---|
| A* | 400 | 20 | 21 |
| A | 400 | 25.5 | 20.5 |
| B* | 200 | 15.1 | 23.2 |
| B | 200 | 18.4 | 11.2 |
| C* | 175 | 22 | 22 |
| C | 175 | 24.5 | 22 |

From Table 2 it can be seen that when the polymer is directly added to the aqueous spray formulation without surfactant (comparative examples A*, B*, and C*), the polymer is immediately available, which is evidenced by a value of V% < 100µm that is constant in time or increasing in time, due to the mechanical degradation of the one or more water-soluble polymers.

Conversely when the polymer composition is added as a dispersion according to the invention to the aqueous spray formulation (examples A, B, and C), samples show values of V% < 100µm that decrease in time due to the controlled release of the one or more water-soluble polymers.

### Example 2

The composition of an oil suspension according to the invention is shown in Table 3. A oil suspension comprising polyethylene oxide with an average molecular weight of 4x10⁶ (four million - PEO) was prepared. The oil suspensions were prepared by mixing the surfactants with Canola oil to obtain an oil phase. Subsequently, the polymer composition was dispersed in the oil phase.

**Table 3**

| | D |
|---|---|
| Polymer type | PEO |
| wt. % polymer in polymer composition | 100 |
| wt. % polymer in suspension | 1.50 |
| wt. % synperonic ETK in suspension | 15 |
| wt. % oil in suspension | 83.50 |
| Aqueous spray formulation g polymer composition / I water | 0.05 |

For the sample in table 3 the volume % of droplets < 100 micrometer and the viscosity were determined as a function of time. These results are shown in Table 4. The results for the sample according to the invention (D) were compared with a solution of the polymer in water, without any surfactant; this is the comparative example and is marked with an "*".

**Table 4**

| Sample | tf (s) | V% < 100µm at to (%) | V% < 100µm at t_{f} (%) | η at t₀ (mPa.s) | η at t_{f} (mPa.s) |
|---|---|---|---|---|---|
| D* | 200 | 15 | 24 | 0.002 | 0.002 |
| D | 200 | 23 | 15 | 0.016 | 0.11 |

From Table 4 it can be seen that when the polymer is directly added to the aqueous spray formulation without surfactant (comparative example D*), the polymer is immediately available. Conversely when the polymer composition is added as an oil suspension according to the invention (example D) to the aqueous spray formulation, the polymer composition exhibits controlled release.

Moreover, controlled released of the polymer is evidenced by an increase of the viscosity of the aqueous spray formulation in time.

The person skilled in the art will understand that if mechanical degradation of the polymer occurs, the viscosity of the aqueous spray formulation will either remain constant or will decrease. From the comparative example it can be seen that in the absence of controlled release, viscosity of the aqueous spray formulation remains constant, i.e. mechanical degradation is not controlled.

### Example 3

The composition of a suspoemulsion according to the invention is shown in Table 5. A suspoemulsion comprising polyethylene oxide with an average molecular weight of 4x10⁶ (four million - PEO) was prepared. The suspoemulsion was prepared by: i) preparing an oil suspension as that prepared in Example D; ii) preparing an aqueous phase by mixing the surfactants with water; and iii) dispersing the oil suspension in the aqueous phase.

**Table 5**

| | E |
|---|---|
| Polymer type | PEO |

| Aqueous phase | |
|---|---|
| wt.% Triton X-100 | 9 |
| wt.% synperonic ETK | 11 |

| Polymer composition | |
|---|---|
| wt. % polymer in polymer composition | 100 |
| wt. % polymer in suspoemulsion | 1 |
| wt. % suspension in suspoemulsion | 75 |
| Aqueous spray formulation g polymer composition / I water | 0.05 |

For the sample in table 5 the volume % of droplets < 100 micrometer and the viscosity were determined as a function of time. These results are shown in Table 6. The results for the sample according to the invention (E) were compared with a solution of the polymer in water, without preparing a suspoemulsion; this is the comparative example and is marked with an "*".

**Table 6**

| Sample | tf (s) | V% < 100µm at t₀ (%) | V% < 100µm at t_{f} (%) | η at t₀ (mPa. s) | η at t_{f} (mPa.s) |
|---|---|---|---|---|---|
| E* | 200 | 15 | 24 | 0.002 | 0.002 |
| E | 200 | 23 | 10 | 0.002 | 0.004 |

From Table 6 it can be seen that when the polymer is directly added to the aqueous spray formulation without preparing the suspoemulsion (comparative example E*), the polymer is immediately available. Conversely, when the polymer composition is added as a suspoemulsion according to the invention (example E) to the aqueous spray formulation, the polymer composition exhibits controlled release.

As previously discussed, controlled released of the polymer is evidenced by an increase of the viscosity of the aqueous spray formulation in time.

The person skilled in the art will understand that if mechanical degradation of the polymer occurs, the viscosity of the aqueous spray formulation will either remain constant or will decrease. From the comparative example it can be seen that in the absence of controlled release, viscosity of the aqueous spray formulation remains constant, i.e. mechanical degradation is not controlled.

### Example 4

The composition of granulates is shown in Table 7. These granulates were prepared following the next steps:
i. providing a powder comprising the one or more water-soluble polymers,
ii. dissolving the at least one non-ionic surfactant in water to obtain an aqueous composition,
iii. spraying the powder of step i. with the aqueous composition of step ii. To produce clusters comprising the one or more water-soluble polymers and the at least one non-ionic surfactant,
iv. drying of the clusters obtained in step iii. to produce a granulate of a polymer composition, said polymer composition comprising one or more water-soluble polymers and at least one non-ionic surfactant.

Granulates were prepared using the following polymers: polyethylene oxide with an average molecular weight of 4×10⁶ (four million - PEO) and Polyacrylamide with an average molecular weight of 5×10⁶ (five million). In addition, for the granulate comprising PEO with an average molecular weight of 4x10⁶ (four million - PEO), polyethylene oxide with an average molecular weight of between 1×10⁵ and 2×10⁵ (one hundred thousand and two hundred thousand - PEO) was also added.

**Table 7**

| | F | G |
|---|---|---|
| Polymer type | PEO | Polyacrylamide |
| wt.% polymer in polymer composition (granulate) | 25 | 77 |
| wt. % polysorbate 20 in polymer composition (granulate) | 25 | 23 |
| wt.% synperonic ^{®} PE/F127 in polymer composition (granulate) | 25 | 0 |
| wt. % PEO with an average molecular weight between 1x10⁵ and 2x10⁵ in polymer composition (granulate) | 25 | 0 |
| Aqueous spray formulation g polymer composition / I water | 0.125 | 0.25 |

For the samples in table 7 the volume % of droplets < 100 micrometer and the viscosity were determined as a function of time. These results are shown in Table 8. The results for samples with controlled release were compared with a solution of the polymers in water, in which the polymers are not in the form of a granulate comprising one or more polymers and a surfactant; these are the comparative examples which are marked with an "*". Example G is not according to the present invention, since it does not comprise polyethylene oxide as water-soluble polymer.

**Table 8**

| Sample | tf (s) | V% < 100µm at t₀ (%) | V% < 100µm at t_{f} (%) | η at t₀ (mPa.s) | η at t_{f} (mPa.s) |
|---|---|---|---|---|---|
| F* | 400 | 14 | 24 | - | - |
| F | 200 | 19 | 9 | 0.009 | 0.016 |
| G* | 200 | 19.5 | 22 | - | - |
| G | 200 | 26 | 21 | - | - |

From Table 8 it can be seen that when the polymer is directly added to the aqueous spray formulation, i.e. not in the form of a granulate comprising one or more water-soluble polymers and at least one surfactant (comparative examples F* and G*), the polymer is immediately available. Conversely when the polymer composition is added as a granulate comprising the one or more water-soluble polymer and a surfactant to the aqueous spray formulation (examples F and G), the polymer composition exhibits controlled release.

As previously discussed, controlled released of the polymer is evidenced by an increase of the viscosity of the aqueous spray formulation in time.

The person skilled in the art will understand that if mechanical degradation of the polymer occurs, the viscosity of the aqueous spray formulation will either remain constant or will decrease. From the comparative example it can be seen that in the absence of controlled release, viscosity of the aqueous spray formulation remains constant, i.e. mechanical degradation is not controlled.

## Claims

1. Dispersion for an aqueous spray formulation with controlled mechanical degradation, said dispersion comprising a polymer composition and at least one liquid phase,
wherein said polymer composition is dispersed in said at least one liquid phase,
wherein said polymer composition comprises one or more water-soluble polymers,
wherein said one or more water-soluble polymers is polyethylene oxide,
wherein said at least one liquid phase is an oil phase and/or an aqueous phase, and
wherein said at least one liquid phase comprises at least one non-ionic surfactant with a HLB of more than 10, preferably of more than 12.

2. Dispersion according to claim 1, wherein said at least one non-ionic surfactant is chosen from the group consisting of surfactants having a lipophilic part and an ethylene oxide-containing hydrophilic part, surfactants having a lipophilic part and a polyethylene-containing hydrophilic part, surfactants having a lipophilic part and a polysaccharide-containing hydrophilic part, and block-copolymer surfactants having a polymer lipophilic part and a polymer hydrophilic part, and combinations thereof.

3. Dispersion according to claim 2, wherein said lipophilic part is a hydrocarbon part, preferably comprised of either polypropylene oxide or one or more aliphatic hydrocarbon chains, preferably alkyl groups or fatty acid residue, more preferably selected from the group consisting of fatty alcohol polyglycol ethers, preferably ethoxylates, or ethylene glycol n-alkane ethers.

4. Dispersion according to claim 2, wherein said block copolymer surfactants are surfactants having one polymer lipophilic part and one polymer hydrophilic part, preferably comprising styrene or PPO as the polymer lipophilic part, more preferably a PEO-PPO block copolymer.

5. Dispersion according to any of the preceding claims, said dispersion further comprising at least one active ingredient, such as a plant protection product, a dye, or a chemical compound for coating a surface, preferably wherein said dispersion comprising 5-70 wt.% of said at least one active ingredient, preferably 10-60 wt.%, more preferably 15-50 wt.%, based on the total weight of the dispersion.

6. Dispersion according to claim 5, wherein said dispersion comprises a polymer composition, said polymer composition comprising 10-70 wt.% of said at least one active ingredient, preferably 15-60 wt.%, more preferably 15-50 wt.%, based on the total weight of the polymer composition, and/or wherein said polymer composition comprises 30-100 wt.% of said one or more water-soluble polymers, preferably 40-100 wt.%, more preferably 50-100 wt.%, based on the total weight of the polymer composition.

7. Dispersion according to any of the claims 1-6, wherein said dispersion is an aqueous suspension of said polymer composition in one liquid phase, wherein said liquid phase is an aqueous phase, preferably wherein said suspension comprises 0.001- 10 wt.% of the one or more water-soluble polymer and 0.1-15 wt.% of said non-ionic surfactant, both based on the total weight of the suspension.

8. Dispersion according to any of the claims 1-7, wherein said dispersion comprises an oil suspension of said polymer composition in a first liquid phase, wherein said first liquid phase is an oil phase, preferably said oil phase comprising one or more vegetable oils, preferably wherein said suspension comprises 0.001-10 wt.% of the one or more water-soluble polymer, and wherein said oil phase comprises 0.1-15 wt.% of said non-ionic surfactant, both based on the total weight of the suspension.

9. Dispersion according to claim 8, said dispersion further comprising a second liquid phase, said suspension of said polymer composition in a first liquid phase being further dispersed in said second liquid phase, said second liquid phase being an aqueous phase, and said dispersion being a suspoemulsion of said suspension of said polymer composition in an oil phase dispersed in an aqueous phase, preferably wherein said aqueous phase comprises 0.1-15 wt.% of said at least one non-ionic surfactant, based on the total weight of the suspoemulsion.

10. Method for controlling the mechanical degradation of a dispersion,
said dispersion being for an aqueous spray formulation,
said dispersion comprising a suspension of a polymer composition dispersed in at least one liquid phase,
wherein said polymer composition comprises one or more water-soluble polymers, wherein said one or more water-soluble polymers is polyethylene oxide,
wherein said at least one liquid phase is an oil phase and/or an aqueous phase,
wherein said at least one liquid phase comprises at least one non-ionic surfactant with a HLB of more than 10, preferably of more than 12; and
wherein said method comprises the steps of:
i) providing the polymer composition,
ii) preparing the at least one liquid phase by mixing the at least one non-ionic surfactant with said at least one liquid phase,
iii) preparing the suspension by dispersing the polymer composition of step i) in the at least one liquid phase of step ii).

11. Method according to claim 10, wherein the liquid phase of step ii) is an aqueous phase.

12. Method according to claim 10, wherein the liquid phase of step ii) is a first liquid phase, said first liquid phase being an oil phase.

13. Method according to claim 12, wherein said method further includes steps:
iv) preparing a second liquid phase, said second liquid phase being an aqueous phase, by mixing at least one non-ionic surfactant with water,
v) preparing a suspoemulsion by dispersing the suspension of step iii) in the aqueous phase of step iv).

14. Use of the dispersion according to any of the claims 1-9 for controlling mechanical degradation of an aqueous spray formulation.

15. Method for preparing an aqueous spray formulation by mixing the dispersion according to any of the claims 1-9 and water in a weight ratio of between 1:50 to 1:1000.

## Patentansprüche

1. Dispersion für eine wässrige Sprühformulierung mit geregeltem mechanischem Abbau, wobei die Dispersion eine Polymerzusammensetzung und wenigstens eine flüssige Phase umfasst,
wobei die Polymerzusammensetzung in der wenigstens einen flüssigen Phase dispergiert ist,
wobei die Polymerzusammensetzung ein oder mehrere wasserlösliche Polymere umfasst,
wobei das eine oder die mehreren wasserlöslichen Polymere Polyethylenoxid sind,
wobei die wenigstens eine flüssige Phase eine Ölphase und/oder eine wässrige Phase ist und
wobei die wenigstens eine flüssige Phase wenigstens ein nichtionisches Tensid mit einem HLB von mehr als 10, vorzugsweise mehr als 12, umfasst.

2. Dispersion gemäß Anspruch 1, wobei das wenigstens eine nichtionische Tensid ausgewählt ist aus der Gruppe bestehend aus Tensiden mit einem lipophilen Teil und einem Ethylenoxid-enthaltenden hydrophilen Teil, Tensiden mit einem lipophilen Teil und einem Polyethylen-enthaltenden hydrophilen Teil, Tensiden mit einem lipophilen Teil und einem Polysaccharid-enthaltenden hydrophilen Teil und Blockcopolymer-Tensiden mit einem lipophilen Polymerteil und einem hydrophilen Polymerteil, und Kombinationen davon.

3. Dispersion gemäß Anspruch 2, wobei der lipophile Teil ein Kohlenwasserstoffteil ist, der vorzugsweise aus Polypropylenoxid oder einer oder mehreren aliphatischen Kohlenwasserstoffketten, vorzugsweise Alkylgruppen oder Fettsäurerest, bevorzugter ausgewählt aus der Gruppe bestehend aus Fettalkohol-Polyglycolethern, vorzugsweise Ethoxylaten oder Ethylenglycol-n-alkanethern, besteht.

4. Dispersion gemäß Anspruch 2, wobei die Blockcopolymer-Tenside Tenside mit einem lipophilen Polymerteil und einem hydrophilen Polymerteil sind, vorzugsweise umfassend Styrol oder PPO als den lipophilen Polymerteil, bevorzugter ein PEO-PPO-Blockcopolymer.

5. Dispersion gemäß einem der vorstehenden Ansprüche, wobei die Dispersion ferner wenigstens einen aktiven Inhaltsstoff umfasst, wie z.B. ein Pflanzenschutzprodukt, einen Farbstoff oder eine chemische Verbindung zum Beschichten einer Oberfläche, wobei die Dispersion vorzugsweise 5-70 Gew.-%, vorzugsweise 10-60 Gew.-%, bevorzugter 15-50 Gew.-%, bezogen auf das Gesamtgewicht der Dispersion, an dem wenigstens einen aktiven Inhaltsstoff umfasst.

6. Dispersion gemäß Anspruch 5, wobei die Dispersion eine Polymerzusammensetzung umfasst, wobei die Polymerzusammensetzung 10-70 Gew.-%, vorzugsweise 15-60 Gew.-%, bevorzugter 15-50 Gew.-%, bezogen auf das Gesamtgewicht der Polymerzusammensetzung, an dem wenigstens einen aktiven Inhaltsstoff umfasst, und/oder wobei die Polymerzusammensetzung 30-100 Gew.-%, vorzugsweise 40-100 Gew.-%, bevorzugter 50-100 Gew.-%, bezogen auf das Gesamtgewicht der Polymerzusammensetzung, an dem einen oder den mehreren wasserlöslichen Polymeren umfasst.

7. Dispersion gemäß einem der Ansprüche 1-6, wobei die Dispersion eine wässrige Suspension der Polymerzusammensetzung in einer flüssigen Phase ist, wobei die flüssige Phase eine wässrige Phase ist, wobei die Suspension vorzugsweise 0,001-10 Gew.-% an dem einen oder den mehreren wasserlöslichen Polymeren und 0,1-15 Gew.-% an dem nichtionischen Tensid umfasst, beides bezogen auf das Gesamtgewicht der Suspension.

8. Dispersion gemäß einem der Ansprüche 1-7, wobei die Dispersion eine Ölsuspension der Polymerzusammensetzung in einer ersten flüssigen Phase umfasst, wobei die erste flüssige Phase eine Ölphase ist, wobei die Ölphase vorzugsweise ein oder mehrere Pflanzenöle umfasst, wobei die Suspension vorzugsweise 0,001-10 Gew.-% an dem einen oder den mehreren wasserlöslichen Polymeren umfasst und wobei die Ölphase 0,1-15 Gew.-% an dem nichtionischen Tensid umfasst, beides bezogen auf das Gesamtgewicht der Suspension.

9. Dispersion gemäß Anspruch 8, wobei die Dispersion ferner eine zweite flüssige Phase umfasst, die Suspension der Polymerzusammensetzung in einer ersten flüssigen Phase ferner in der zweiten flüssigen Phase dispergiert ist, die zweite flüssige Phase eine wässrige Phase ist und die Dispersion eine Suspoemulsion der Suspension der Polymerzusammensetzung in einer Ölphase, dispergiert in einer wässrigen Phase, ist, wobei die wässrige Phase vorzugsweise 0,1-15 Gew.-% an dem wenigstens einen nichtionischen Tensid, bezogen auf das Gesamtgewicht der Suspoemulsion, umfasst.

10. Verfahren zur Regelung des mechanischen Abbaus einer Dispersion,
wobei die Dispersion für eine wässrige Sprühformulierung ist,
wobei die Dispersion eine Suspension einer Polymerzusammensetzung, die in wenigstens einer flüssigen Phase dispergiert ist, umfasst,
wobei die Polymerzusammensetzung ein oder mehrere wasserlösliche Polymere umfasst, wobei das eine oder die mehreren wasserlöslichen Polymere Polyethylenoxid sind,
wobei die wenigstens eine flüssige Phase eine Ölphase und/oder eine wässrige Phase ist,
wobei die wenigstens eine flüssige Phase wenigstens ein nichtionisches Tensid mit einem HLB von mehr als 10, vorzugsweise mehr als 12, umfasst; und
wobei das Verfahren die Schritte umfasst:
i) Bereitstellen der Polymerzusammensetzung,
ii) Herstellen der wenigstens einen flüssigen Phase durch Mischen des wenigstens einen nichtionischen Tensids mit der wenigstens einen flüssigen Phase,
iii) Herstellen der Suspension durch Dispergieren der Polymerzusammensetzung von Schritt i) in der wenigstens einen flüssigen Phase von Schritt ii).

11. Verfahren gemäß Anspruch 10, wobei die flüssige Phase von Schritt ii) eine wässrige Phase ist.

12. Verfahren gemäß Anspruch 10, wobei die flüssige Phase von Schritt ii) eine erste flüssige Phase ist, wobei die erste flüssige Phase eine Ölphase ist.

13. Verfahren gemäß Anspruch 12, wobei das Verfahren ferner die Schritte umfasst:
iv) Herstellen einer zweiten flüssigen Phase, wobei die zweite flüssige Phase eine wässrige Phase ist, durch Mischen wenigstens eines nichtionischen Tensids mit Wasser,
v) Herstellen einer Suspoemulsion durch Dispergieren der Suspension von Schritt iii) in der wässrigen Phase von Schritt iv).

14. Verwendung der Dispersion gemäß einem der Ansprüche 1-9 zur Regelung von mechanischem Abbau einer wässrigen Sprühformulierung.

15. Verfahren zur Herstellung einer wässrigen Sprühformulierung durch Mischen der Dispersion gemäß einem der Ansprüche 1-9 und Wasser in einem Gewichtsverhältnis von zwischen 1:50 und 1:1000.

## Revendications

1. Dispersion pour une formulation de pulvérisation aqueuse à dégradation mécanique contrôlée, ladite dispersion comprenant une composition polymère et au moins une phase liquide,
dans laquelle ladite composition polymère est dispersée dans ladite au moins une phase liquide,
dans laquelle ladite composition polymère comprend un ou plusieurs polymères solubles dans l'eau,
dans laquelle ledit ou lesdits polymères solubles dans l'eau sont un oxyde de polyéthylène,
dans laquelle ladite au moins une phase liquide est une phase huileuse et/ou une phase aqueuse, et
dans laquelle ladite au moins une phase liquide comprend au moins un tensioactif non ionique avec un HLB supérieur à 10, de préférence supérieur à 12.

2. Dispersion selon la revendication 1, dans laquelle ledit au moins un tensioactif non ionique est choisi dans le groupe consistant en des tensioactifs ayant une partie lipophile et une partie hydrophile contenant de l'oxyde d'éthylène, des tensioactifs ayant une partie lipophile et une partie hydrophile contenant du polyéthylène, des tensioactifs ayant une partie lipophile et une partie hydrophile contenant du polysaccharide, et des tensioactifs copolymères blocs ayant une partie lipophile polymère et une partie hydrophile polymère, et leurs combinaisons.

3. Dispersion selon la revendication 2, dans laquelle ladite partie lipophile est une partie hydrocarbonée, de préférence constituée soit d'un oxyde de polypropylène, soit d'une ou plusieurs chaînes hydrocarbonées aliphatiques, de préférence des groupes alkyle ou un résidu d'acide gras, plus préférablement choisie dans le groupe consistant en des éthers polyglycoliques d'alcools gras, de préférence des éthoxylates, ou des éthers n-alcanes d'éthylène glycol.

4. Dispersion selon la revendication 2, dans laquelle lesdits tensioactifs copolymères blocs sont des tensioactifs ayant une partie polymère lipophile et une partie polymère hydrophile, comprenant de préférence du styrène ou du PPO comme partie polymère lipophile, plus préférablement un copolymère bloc PEO-PPO.

5. Dispersion selon l'une des revendications précédentes, ladite dispersion comprenant en outre au moins un ingrédient actif, tel qu'un produit phytosanitaire, un colorant ou un composé chimique pour revêtir une surface, de préférence dans laquelle ladite dispersion comprenant 5 à 70% en poids dudit au moins un ingrédient actif, de préférence 10 à 60% en poids, plus préférablement 15 à 50% en poids, sur la base du poids total de la dispersion.

6. Dispersion selon la revendication 5, dans laquelle ladite dispersion comprend une composition polymère, ladite composition polymère comprenant 10 à 70% en poids dudit au moins un ingrédient actif, de préférence 15 à 60% en poids, plus préférablement 15 à 50% en poids, sur la base du poids total de la composition polymère, et/ou dans laquelle ladite composition polymère comprend 30 à 100% en poids dudit ou desdits polymères solubles dans l'eau, de préférence 40 à 100% en poids, plus préférablement 50 à 100% en poids, sur la base du poids total de la composition polymère.

7. Dispersion selon l'une des revendications 1 à 6, dans laquelle ladite dispersion est une suspension aqueuse de ladite composition polymère dans une phase liquide, dans laquelle ladite phase liquide est une phase aqueuse, de préférence dans laquelle ladite suspension comprend 0,001 à 10% en poids du ou des plusieurs polymères solubles dans l'eau et 0,1 à 15% en poids dudit tensioactif non ionique, tous deux sur la base du poids total de la suspension.

8. Dispersion selon l'une des revendications 1 à 7, dans laquelle ladite dispersion comprend une suspension huileuse de ladite composition polymère dans une première phase liquide, dans laquelle ladite première phase liquide est une phase huileuse, de préférence ladite phase huileuse comprenant une ou plusieurs huiles végétales, de préférence dans laquelle ladite suspension comprend 0,001 à 10% en poids du ou des plusieurs polymères solubles dans l'eau, et dans laquelle ladite phase huileuse comprend 0,1 à 15% en poids dudit tensioactif non ionique, tous deux sur la base du poids total de la suspension.

9. Dispersion selon la revendication 8, ladite dispersion comprenant en outre une deuxième phase liquide, ladite suspension de ladite composition polymère dans une première phase liquide étant en outre dispersée dans ladite deuxième phase liquide, ladite deuxième phase liquide étant une phase aqueuse, et ladite dispersion étant une suspo-émulsion de ladite suspension de ladite composition polymère dans une phase huileuse dispersée dans une phase aqueuse, de préférence dans laquelle ladite phase aqueuse comprend 0,1 à 15% en poids dudit au moins un tensioactif non ionique, sur la base du poids total de la suspo-émulsion.

10. Procédé de contrôle de la dégradation mécanique d'une dispersion,
ladite dispersion étant destinée pour une formulation de pulvérisation aqueuse,
ladite dispersion comprenant une suspension d'une composition polymère dispersée dans au moins une phase liquide,
dans lequel ladite composition polymère comprend un ou plusieurs polymères solubles dans l'eau, où ledit ou lesdits polymères solubles dans l'eau sont un oxyde de polyéthylène,
dans lequel ladite au moins une phase liquide est une phase huileuse et/ou une phase aqueuse,
dans lequel ladite au moins une phase liquide comprend au moins un tensioactif non ionique avec un HLB supérieur à 10, de préférence supérieur à 12 ; et
dans lequel ledit procédé comprend les étapes de :
i) fourniture de la composition polymère,
ii) préparation de l'au moins une phase liquide en mélangeant l'au moins un tensioactif non ionique avec ladite au moins une phase liquide,
iii) préparation de la suspension en dispersant la composition polymère de l'étape i) dans l'au moins une phase liquide de l'étape ii).

11. Procédé selon la revendication 10, dans lequel la phase liquide de l'étape ii) est une phase aqueuse.

12. Procédé selon la revendication 10, dans lequel la phase liquide de l'étape ii) est une première phase liquide, ladite première phase liquide étant une phase huileuse.

13. Procédé selon la revendication 12, dans lequel ledit procédé comporte en outre les étapes de :
iv) préparation d'une deuxième phase liquide, ladite deuxième phase liquide étant une phase aqueuse, en mélangeant au moins un tensioactif non ionique avec de l'eau,
v) préparation d'une suspo-émulsion en dispersant la suspension de l'étape iii) dans la phase aqueuse de l'étape iv).

14. Utilisation de la dispersion selon l'une quelconque des revendications 1 à 9 pour contrôler la dégradation mécanique d'une formulation de pulvérisation aqueuse.

15. Procédé de préparation d'une formulation de pulvérisation aqueuse en mélangeant la dispersion selon l'une quelconque des revendications 1 à 9 et de l'eau dans un rapport pondéral compris entre 1:50 et 1:1000.
